# EUROPÄISCHE PATENTSCHRIFT

(11) **EP 2 044 845 B1**
(45) Veröffentlichungstag und Bekanntmachung des Hinweises auf die Patenterteilung: **14.01.2015**
(21) Anmeldenummer: 07019471.7
(22) Anmeldetag: 04.10.2007
(51) Int. Cl.: C12P 7/06, A23C 21/02, A23L 1/09, A23L 1/304, A23C 9/144, C13B 20/18

(54) **Verfahren zur Aufarbeitung von Melasse**
Method for processing molasses
Procédé de préparation de mélasse

(43) Veröffentlichungstag der Anmeldung: 08.04.2009
(60) Teilanmeldung: 13005082.6
(73) Patentinhaber: Molkerei Alois Müller GmbH & Co. KG, 86850 Aretsried (DE)
(72) Erfinder: Cloidt, Roland, 01454 Radeberg (DE); Lehmann, Hanno, 59846 Sundern (DE)
(74) Vertreter: Leinweber & Zimmermann

(56) Entgegenhaltungen:
- EP-A1- 1 869 984
- DE-A1- 2 047 139
- GB-A- 1 575 089
- US-A- 2 584 158
- US-A- 4 138 501
- CARIC MARIJANA: "Concentrated and dried dairy products , Lactose" CONCENTRATED AND DRIED DAIRY PRODUCTS, VCH PUBLISHERS, NEW YORK, NY, US, 1991, Seiten 227-234, XP002410105

## Beschreibung

Die Erfindung betrifft ein Verfahren zur Aufarbeitung von Melasse mit den Verfahrensschritten: Einstellen des Trockenmassegehalts der dem Verfahren zugeführten Melasse auf 10% oder weniger, Isolieren einer mineralisch abgereicherten Komponente durch Wasserentzug durch Eindampfen, Kristallieren der aufkonzentrierten Komponente, Abtrennen der durch das Kristallisieren ausgefällten kristallinen Phase von der flüssigen Phase, Waschen der abgetrennten kristallinen Phase mit einer Waschflüssigkeit, die aus einem Molkeaufbereitungsverfahren entnommen wird, bei dem der Molke zuerst Eiweiß durch ein Membrantrennverfahren und anschließend Laktose durch ein Kristallisationsverfahren entzogen wird, dessen Mutterlauge die Melasse liefert und die einen Trockenmassegehalt zwischen 18 und 35% aufweist, und die Einstellung des Trockenmassegehalts durch Zugabe von in dem Eindampfschritt entstehenden Brüden erfolgt.

Melasse fällt insbesondere bei der Aufarbeitung von Molke an, bei der die Molke zunächst durch ein Membranverfahren in eine proteinangereicherte Retentatkomponente und eine laktoseangereicherte Permeatkomponente getrennt wird. Aus letzterer wird nach Eindampfen und Kristallisieren durch mechanische Trennverfahren Laktose abgetrennt. Bei dieser Abtrennung bleibt die Melasse als unverwertete Komponente zurück, die bei herkömmlichen Verfahren entsorgt werden musste.

Bei einem Verfahren der eingangs genannten Art (US-A-2 584 158) erfolgt die Abtrennung von Mineralien in Anionen- und Kationenaustauschern. Dazu wird die dem Austauscher zugeführte Melasse mit in einem vorangegangenen Regenerationszyklus des Austauschers angefallenen Waschwasser und zusätzlich zugeführtem Wasser verdünnt.

Die nicht vorveröffentlichte EP 1 869 984 A1 offenbart ein Verfahren zur Verarbeitung von Molke, bei dem der Molke durch Ultrafiltration Protein entzogen und durch Abtrennen von Mineralien durch Elektrodialyse oder ein Ionenaustauschverfahren eine mineralisch abgereicherte Komponente isoliert wird. Diese wird durch Wasserentzug aufkonzentriert und Laktose auskristallisiert. Letztere wird abgetrennt und gereinigt.

Der Erfindung liegt die Aufgabe zugrunde, ein Verfahren zur Aufarbeitung von Melasse der eingangs genannten Art zu schaffen, durch das wertvolle Inhaltsstoffe der Melasse gewonnen werden können.

Erfindungsgemäß wird diese Aufgabe dadurch gelöst, dass das Abtrennen von Mineralien aus der Melasse durch Elektrodialyse und die Einstellung des Trockenmassegehalts durch Zugabe von in dem Eindampfschritt entstehenden Brüden erfolgt.

Die dem Verfahren aus der Molkeverarbeitung zugeführte Melasse weist einen Trockenmassegehalt von 18 bis 35% auf. Der verringerte Mineralgehalt der durch das Abtrennen von Mineralien isolierten, mineralisch abgereicherten Komponente ist für deren nachfolgende Kristallisation vorteilhaft.

Für die Mineralabtrennung im technischen Maßstab eignet sich die Elektrodialyse, mit der es gelingt, 50 bis 80%, insbesondere 60 bis 65%, der in der Melasse befindlichen Mineralien abzutrennen. Für dieses Verfahren ist es dabei vorteilhaft, den Trockenmassegehalt der dem Verfahren zugeführten Melasse auf z.B. 10% einzustellen. Dies kann dadurch geschehen, dass der dem Verfahren zugeführten Melasse Brüden zugesetzt werden, die in nachfolgenden Verfahrensschritten entstehen. Dadurch kann die Wasserbilanz des Gesamtprozesses ausgeglichen werden, so dass kein zusätzliches Frischwasser zugeführt werden muss.

Die durch die Elektrodialyse isolierte, mineralisch abgereicherte Komponente weist beispielsweise einen Trockenmassegehalt von 20% auf. In der Trockenmasse beträgt der Aschegehalt beispielsweise 8 bis 9% sowie der Gehalt an Glucose und Milchsäure 2 bis 5%.

Durch das Aufkonzentrieren der mineralisch abgereicherten Komponente, das insbesondere durch Eindampfen erfolgt, wird der Trockenmassegehalt erhöht, beispielsweise auf 60 bis 70%.

Das Kristallisieren der solchermaßen aufkonzentrierten Komponente erfolgt vorzugsweise in einem Kristallisationstank über eine Zeitdauer von beispielsweise 25 bis 35 Stunden.

Die Suspension, einschließlich der ausgefällten kristallinen Phase, kann dann entweder entsprechend der Patentanmeldung 06 012 916.0-2114 - "Verfahren zur Herstellung von Permeatpulver" - behandelt werden oder entsprechend folgendem Prozeßablauf:
Die Suspension einschließlich der kristallinen Phase wird in einer mehrstufigen, vorzugsweise einer zwei- bis vierstufigen, Dekantier- und Waschstraße von allen unerwünschten Inhaltsstoffen befreit. In der solchermaßen abgereicherten Komponente beträgt der Gehalt an Glucose und Milchsäure, die für eine Trocknung ungünstig sind, in der Trockenmasse beispielsweise weniger als 1 %, vorzugsweise weniger als 0,2 %. Somit kann diese abgereicherte Komponente wirkungsvoll einem Trocknungsvorgang unterzogen werden, dessen Endprodukt ein Laktosepulver ist.

Mit besonderem Vorteil wird die von der kristallinen Phase getrennte flüssige Phase des Kristallisationsprozesses und/oder die die abgetrennte Glucose und Milchsäure enthaltende Komponente und/oder die die abgetrennten Mineralien enthaltende Komponente zu Ethanol weiterverarbeitet. Dies ist Gegenstand der abhängigen Ansprüche 22 bis 30.

In der folgenden Beschreibung wird ein Ausführungsbeispiel der Erfindung anhand der Zeichnung näher erläutert. Hierin zeigen:
- Fig. 1: ein Ablaufschema eines Gesamtprozesses, und
- Fig. 2: Einzelheiten eines der Ethanolherstellung dienenden Zweiges des Gesamtprozesses von Fig. 1.

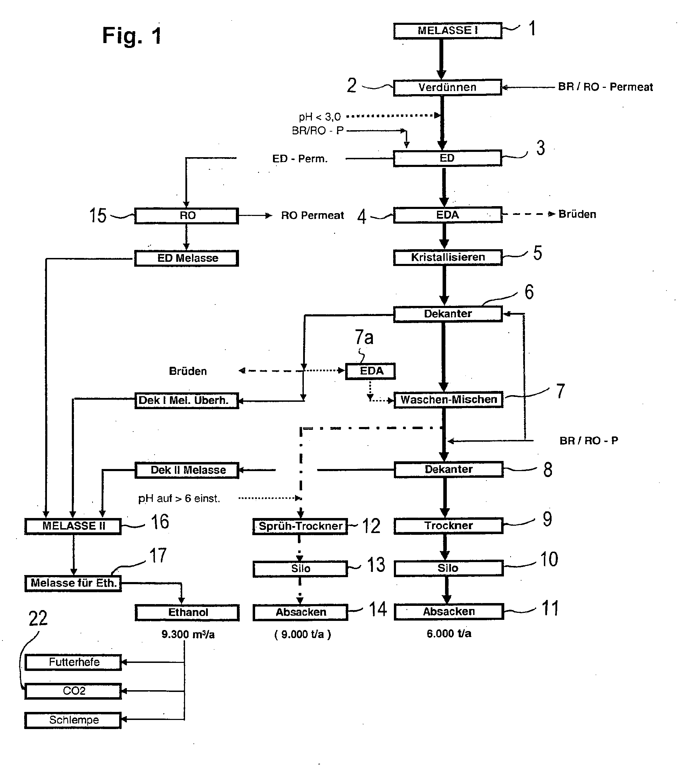

In dem Ablaufschema von Fig. 1 symbolisiert ein Block 1 die eingangsseitige Zuführung von Melasse zum Gesamtprozeß, wobei diese Melasse am Ende eines Laktoseprozesses anfällt, bei dem Molke zuerst eine proteinangereicherte Retentatkomponente entzogen und von der verbleibenden laktosereichen Permeatkomponente durch Eindampfen und Kristallisieren Laktose abgetrennt wird. In einer im Verfahrensablauf folgenden Stufe 2 wird die bei 1 zugeführte Melasse, die einen Trockenmassegehalt von etwa 25 % aufweisen kann, durch Verdünnen auf einen Trockenmassegehalt von 10 % oder kleiner standardisiert. Als Verdünnungsmittel für diese Reduzierung des Trockenmassegehaltes dienen Brüden und/oder ein Permeat aus reverser Osmose, die in noch zu erläuternden nachgeschalteten Prozeßstufen anfallen.

Der pH-Wert der solchermaßen standardisierten Melasse kann durch Zugabe von Hilfsstoffen, beispielsweise Salzsäure, auf einen pH-Wert zwischen 2 und 6 eingestellt und von der ggf. eingestellten Melasse in einer auf die Stufe 2 folgenden Stufe 3 durch Elektrodialyse ein wesentlicher Anteil der in der standardisierten Melasse enthaltenen Mineralien, beispielsweise 60 bis 65 %, abgetrennt werden. Es ist geraten, der Elektrodialyse eine mechanische Klärung, vorzugsweise einen Separator oder eine Mikrofiltration, vorzuschalten, um Partikel zurückzuhalten, die die Funktion der Elektrodialyse negativ beeinflussen würden. Die nach der Elektrodialyse verbleibende, mineralisch abgereicherte Komponente, deren Trockenmassegehalt beispielsweise etwa 20 % beträgt, wird in einer auf die Elektrodialysestufe 3 folgenden Eindampferstufe 4 auf eine Konzentration von beispielsweise 65 % Trockenmasse eingedampft und anschließend in einem Kristallisationstank 5 über beispielsweise 25 bis 35 Stunden kristallisiert.

In der dem Kristallisationstank 5 nachgeschalteten mehrstufigen Dekantier- und Waschstraße (Stufen 6, 7 und 8) werden die für die Trocknung unerwünschten Gehalte an Mineralien, Proteinen, Glucose und Milchsäure weitestgehend abgetrennt. Die abgereicherte Komponente aus dem Dekanter 8, deren Gehalt an unerwünschten Inhaltsstoffen beispielsweise kleiner als 1 %, vorzugsweise in Summe kleiner als 0,5 % ist, wird in einer dem Dekanter 8 nachgeschalteten Trocknungsstufe 9 getrocknet, wodurch als Endprodukt ein Laktosepulver entsteht, das in einem Silo 10 zwischengelagert und an einem Ausgang 11 des Silos 10 abgesackt werden kann.

Vorzugsweise sind die Prozesse so zu gestalten, daß in Abhängigkeit vom Markt Permeatpulver oder das höherwertige Laktosepulver hergestellt werden kann. Hierzu kann das in der Waschmischstufe 7 erhaltene Gemisch ganz oder teilweise nach einer pH-Wert-Einstellung auf 6 oder höher in einem Sprühtrockner 12 getrocknet werden, wodurch ein Permeatpulver entsteht, das in einem Silo 13 zwischengelagert und an einem Ausgang 14 des Silos 13 abgesackt werden kann.

Die in den Eindampferstufen 4 und 7a entstehenden Brüden werden zu der Verdünnungsstufe 2, der Elektrodialysestufe 3 sowie den Dekanterstufen 6 und 8 zurückgeführt. Dadurch kann der in den Stufen 1 bis 8 ablaufende Prozeß ohne zusätzliche Frischwasserzufuhr betrieben werden.

Die in der Elektrodialysestufe 3 bei der Gewinnung der mineralisch abgereicherten Komponente verbleibende Komponente wird in einer Stufe 15 durch reverse Osmose aufkonzentriert und das aufkonzentrierte Retentat einem Ethanolgewinnungsprozeß zugeführt. Das Permeat der Stufe 15 wird für einen Bilanzausgleich des Gesamtprozesses zu der Verdünnungsstufe 2, der Elektrodialysestufe 3 und den Dekanterstufen 6 und 8 zurückgeführt.

Dem Ethanolgewinnungsprozeß wird ferner der nicht für die Wasch-Mischstufe 7 verbrauchte Anteil der flüssigen Phase aus dem Dekanter 6 zugeführt. Dieser Anteil beträgt 70 bis 90 %, insbesondere 85 %, und führt insbesondere amorphe Laktose und Galaktose aus dem in den Stufen 1 bis 8 ablaufenden Prozeß.

Weiterhin wird dem Ethanolgewinnungsprozeß die in dem Dekanter 8 abgetrennte flüssige Phase zugeführt. Die Zusammenfassung der drei Teilströme aus der Elektrodialysestufe 3, der Dekanterstufe 6 und der Dekanterstufe 8 zu einem für die Ethanolgewinnung verwendeten Gesamtstrom ist in Fig. 1 durch die Blöcke 16 und 17 veranschaulicht.

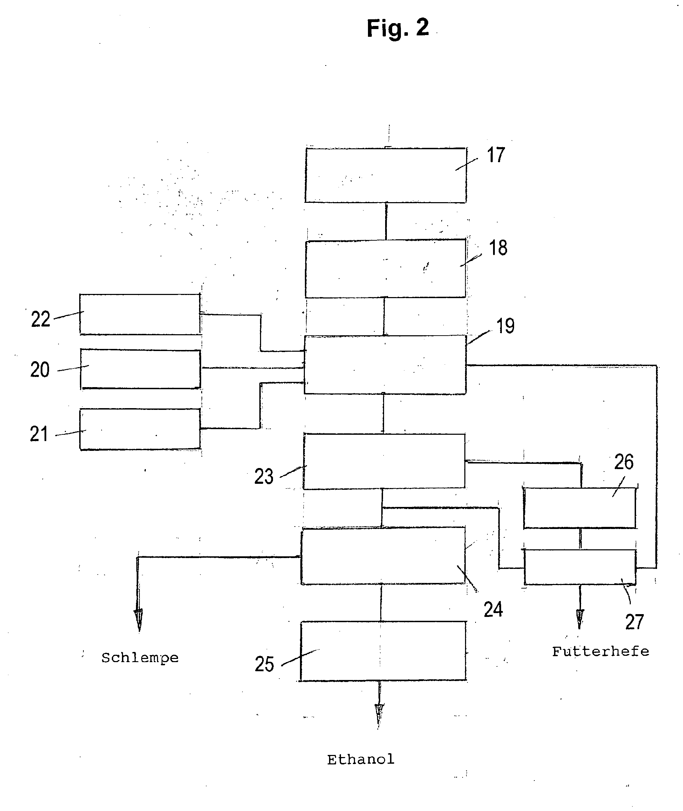

Gemäß Fig. 2 wird der in dem Block 17, bei dem es sich um einen Tank handeln kann, anfallende Gesamtstrom einem Erhitzer 18 zugeführt, in dem eine Erhitzung auf beispielsweise 85° zur Keimabtötung stattfindet. In einem dem Erhitzer 18 nachgeschalteten Fermentationstank 19 wird aus einem Kulturtank 20 Hefe zugesetzt und die Fermentation bei einer Fermentationstemperatur im Bereich vom 25 bis 35° C durchgeführt. Es werden vorzugsweise Hefen der Gattung Kluyveromyces verwendet. Die Einstellung eines geeigneten pH-Wertes, beispielsweise von 4 bis 6, erfolgt durch Zufuhr von Lauge oder Säure aus einem Vorlagebehälter 21. Das bei der Fermentation entstehende CO₂ kann in einer CO₂-Verflüssigungsanlage 22 aufgefangen werden.

Nach abgeschlossener Fermentierung wird das fermentierte Substrat in einem Separator 23 in ein Hefenkonzentrat und ein hefefreies Fermentat getrennt. Aus letzterem wird in einer Stufe 24 Rohalkohol durch Destillieren, Rektifizieren erzeugt. Dieser Alkohol mit bis zu 96 % wird in einer nachgeschalteten Dehydrationsstufe 25 beispielsweise bis auf eine Reinheit von mindestens 99,8 % entwässert, wodurch handelsfähiges Ethanol erhalten wird.

Das in dem Separator 23 abgetrennte Hefenkonzentrat wird in einer Wascheinrichtung 26 mit Wasser versetzt und anschließend in einem zweiten Separator 27 aufkonzentriert. Die in dem Separator 27 abgetrennte Klarphase wird dem der Stufe 24 zulaufenden Fermentat aus dem Separator 23 beigemischt, um enthaltene Restalkohole zurückzugewinnen. Das in dem Düsenseparator 27 abgetrennte Hefenkonzentrat wird bis zu 50 % in den Fermentationstank 19 zurückgeführt. Das nicht zurückgeführte Hefenkonzentrat wird als Futterhefe entnommen. Die beim Destillieren/Rektifizieren in der Stufe 24 anfallende Schlempe wird entnommen und kann zur weiteren Verwendung durch Eindampfen auf 20 bis 30 % Trockenmasse aufkonzentriert werden.

In Fig. 1 sind beispielhaft Jahresmengen für eine Prozeßführung im industriellen Maßstab angegeben. Danach werden ausgehend von einer jährlichen Zuführung von 29.986 t Melasse jährlich 6.000 t Laktosepulver, alternativ 9.000 t Permeatpulver und 9.300 m³ Ethanol gewonnen.

## Patentansprüche

1. Verfahren zur Aufarbeitung von Melasse, die aus einem Molkeaufbereitungsverfahren entnommen wird, bei dem der Molke zuerst Eiweiß durch ein Membrantrennverfahren und anschließend Laktose durch ein Kristallisationsverfahren entzogen wird, dessen Mutterlauge die Melasse liefert und die einen Trockenmassegehalt zwischen 18 und 35% aufweist, mit den Verfahrensschritten:
Einstellen des Trockenmassegehalts der dem Verfahren zugeführten Melasse auf 10% oder weniger,
Isolieren einer mineralisch abgereicherten Komponente durch Abtrennen von Mineralien aus der Melasse,
Aufkonzentrieren der mineralisch abgereicherten Komponente durch Wasserentzug durch Eindampfen,
Kristallisieren der aufkonzentrierten Komponente,
Abtrennen der durch das Kristallisieren ausgefällten kristallinen Phase von der flüssigen Phase,
Waschen der abgetrennten kristallinen Phase mit einer Waschflüssigkeit,
**dadurch gekennzeichnet, dass** das Abtrennen von Mineralien aus der Melasse durch Elektrodialyse und die Einstellung des Trockenmassegehalts durch Zugabe von in dem Eindampfschritt entstehenden Brüden erfolgt.

2. Verfahren nach Anspruch 1, **dadurch gekennzeichnet, dass** das Kristallisieren in einem Kristallisationstank erfolgt.

3. Verfahren nach einem der Ansprüche 1 bis 2, **dadurch gekennzeichnet, dass** das Abtrennen der kristallinen Phase in einem Dekanter erfolgt.

4. Verfahren nach einem der Ansprüche 1 bis 3, **dadurch gekennzeichnet, dass** die Waschflüssigkeit aus der flüssigen Phase des Kristallisationsprozesses entnommen wird.

5. Verfahren nach Anspruch 4, **dadurch gekennzeichnet, dass** die entnommene flüssige Phase zur Bildung der Waschflüssigkeit aufkonzentriert wird.

6. Verfahren nach Anspruch 5, **dadurch gekennzeichnet, dass** das Aufkonzentrieren der entnommenen flüssigen Phase durch Eindampfen erfolgt.

7. Verfahren nach einem der Ansprüche 1 bis 6, **dadurch gekennzeichnet, dass** die aufkonzentrierte Komponente einen Trockenmassegehalt zwischen 60 und 70% aufweist.

8. Verfahren nach einem der Ansprüche 1 bis 7, **dadurch gekennzeichnet, dass** durch die Abtrennung zwischen 60 und 65% der in der zugeführten Melasse enthaltenen Mineralien abgetrennt werden.

9. Verfahren nach einem der Ansprüche 1 bis 8, **dadurch gekennzeichnet, dass** das Kristallisieren über eine Zeitdauer von 25 bis 35 Stunden ausgeführt wird.

10. Verfahren nach einem der Ansprüche 1 bis 9, **dadurch gekennzeichnet, dass** für das Isolieren der mineralisch abgereicherten Komponente der pH-Wert auf kleiner 6,0 eingestellt wird.

11. Verfahren nach einem der Ansprüche 1 bis 10, **dadurch gekennzeichnet, dass** aus der gewaschenen kristallinen Phase durch Abtrennen eine an Glucose und Milchsäure abgereicherte Komponente gewonnen wird.

12. Verfahren nach Anspruch 11, **dadurch gekennzeichnet, dass** das Abtrennen in einem Dekanter erfolgt.

13. Verfahren nach Anspruch 11 oder 12, **dadurch gekennzeichnet, dass** die an Glucose und Milchsäure abgereicherte Komponente getrocknet wird.

14. Verfahren nach einem der Ansprüche 1 bis 10, **dadurch gekennzeichnet, dass** die gewaschene kristalline Phase sprühgetrocknet wird.

15. Verfahren nach Anspruch 14, **dadurch gekennzeichnet, dass** der pH-Wert der gewaschenen kristallinen Phase auf 6 oder höher eingestellt wird.

## Claims

1. A method for processing molasses which are taken from a whey preparation process, wherein first of all albumen is extracted from the whey by a membrane separation method and then lactose is extracted by a crystallisation method, the mother lye of which provides the molasses and which has a dry mass content of between 18 and 35%, comprising the procedural steps:
setting the dry mass content of the molasses introduced to the process to 10% or less,
isolating a mineral-depleted component by separating minerals from the molasses,
concentrating the mineral-depleted component by eliminating water by evaporation,
crystallising the concentrated component,
separating the crystalline phase precipitated by the crystallisation from the liquid phase,
washing the separated crystalline phase with a washing liquid,
**characterised in that** the minerals are separated from the molasses by electrodialysis and by setting the dry mass content by adding vapours produced during the evaporation step.

2. The method according to Claim 1, **characterised in that** the crystallisation takes place in a crystallisation tank.

3. The method according to either of Claims 1 or 2, **characterised in that** the crystalline phase is separated in a decanter.

4. The method according to any of Claims 1 to 3, **characterised in that** the washing liquid is extracted from the liquid phase of the crystallisation process.

5. The method according to Claim 4, **characterised in that** the extracted liquid phase is concentrated to form the washing liquid.

6. The method according to Claim 5, **characterised in that** the extracted liquid phase is concentrated by evaporation.

7. The method according to any of Claims 1 to 6, **characterised in that** the concentrated component has a dry mass content of between 60 and 70%.

8. The method according to any of Claims 1 to 7, **characterised in that** by means of the separation, between 60 and 65% of the minerals contained in the molasses supplied are separated.

9. The method according to any of Claims 1 to 8, **characterised in that** the crystallisation is performed over a period of 25 to 35 hours.

10. The method according to any of Claims 1 to 9, **characterised in that** the pH value is set to less than 6.0 in order to isolate the mineral-depleted component.

11. The method according to any of Claims 1 to 10, **characterised in that** a glucose- and lactic acid-depleted component is obtained from the washed crystalline phase by separation.

12. The method according to Claim 11, **characterised in that** the separation takes place in a decanter.

13. The method according to Claim 11 or 12, **characterised in that** the glucose- and lactic acid-depleted component is dried.

14. The method according to any of Claims 1 to 10, **characterised in that** the washed crystalline phase is spray dried.

15. The method according to Claim 14, **characterised in that** the pH value of the washed crystalline phase is set to 6 or higher.

## Revendications

1. Procédé de préparation de mélasse issue d'un procédé de traitement du lactosérum, dans lequel le lactosérum est débarrassé de ses protéines selon un procédé de séparation sur membrane puis de son lactose selon un procédé de cristallisation, dont la liqueur mère fournit la mélasse et qui présente une teneur en matière sèche située entre 18 et 35 %, comportant les étapes de procédé consistant à :
établir la teneur en matière sèche de la mélasse introduite dans le procédé à 10 % ou moins,
isoler une composante appauvrie en minéraux par séparation de minéraux de la mélasse,
concentrer la composante appauvrie en minéraux par élimination d'eau par le biais d'une évaporation,
cristalliser la composante concentrée,
effectuer une séparation entre la phase cristallisée issue de la cristallisation et la phase liquide,
laver la phase cristallisée séparée à l'aide d'un liquide de lavage,
**caractérisé en ce que** la séparation de minéraux de la mélasse s'effectue par électrodialyse et
l'établissement de la teneur en matière sèche s'effectue par ajout de vapeurs produites lors de l'étape d'évaporation.

2. Procédé selon la revendication 1, **caractérisé en ce que** la cristallisation s'effectue dans une cuve de cristallisation.

3. Procédé selon l'une des revendications 1 à 2, **caractérisé en ce que** la séparation de la phase cristalline s'effectue dans une cuve de décantation.

4. Procédé selon l'une des revendications 1 à 3, **caractérisé en ce que** le liquide de lavage provient de la phase liquide du processus de cristallisation.

5. Procédé selon la revendication 4, **caractérisé en ce que** la phase liquide prélevée est concentrée pour former le liquide de lavage.

6. Procédé selon la revendication 5, **caractérisé en ce que** la concentration de la phase liquide prélevée s'effectue par évaporation.

7. Procédé selon l'une des revendications 1 à 6, **caractérisé en ce que** la composante concentrée présente une teneur en matière sèche entre 60 et 70 %.

8. Procédé selon l'une des revendications 1 à 7, **caractérisé en ce que** la séparation permet de séparer entre 60 et 65 % des minéraux contenus dans la mélasse introduite.

9. Procédé selon l'une des revendications 1 à 8, **caractérisé en ce que** la cristallisation est mise en oeuvre pendant une période de 25 à 35 heures.

10. Procédé selon l'une des revendications 1 à 9, **caractérisé en ce que**, pour l'isolation de la composante appauvrie en minéraux, la valeur du pH est établie à moins de 6,0.

11. Procédé selon l'une des revendications 1 à 10, **caractérisé en ce que** la séparation de la phase cristalline lavée permet d'extraire une composante appauvrie en glucose et en acide lactique.

12. Procédé selon la revendication 11, **caractérisé en ce que** la séparation s'effectue dans une cuve de décantation.

13. Procédé selon la revendication 11 ou 12, **caractérisé en ce que** la composante appauvrie en glucose et en acide lactique est séchée.

14. Procédé selon l'une des revendications 1 à 10, **caractérisé en ce que** la phase cristalline lavée est séchée par pulvérisation.

15. Procédé selon la revendication 14, **caractérisé en ce que** la valeur du pH de la phase cristalline lavée est établie à 6 ou plus.
